# FASCICULE DE BREVET EUROPEEN

(11) **EP 2 735 295 B1**
(45) Date de publication et mention de la délivrance du brevet: **18.11.2015**
(21) Numéro de dépôt: 13193483.8
(22) Date de dépôt: 19.11.2013
(51) Int. Cl.: A61H 7/00, A61H 15/00, A61H 15/02, A61H 23/02, A61N 5/06, A61N 1/30, A45D 40/26, A45D 34/00, A45D 40/00

(54) **Appareil de massage équipe d'un système de distribution de produits cosmétiques**
Massagegerät, das mit einem System zur Verteilung von Kosmetikprodukten ausgestattet ist
Massage apparatus provided with a system for dispensing cosmetic products

(30) Priorité: 22.11.2012 FR 1261106
(43) Date de publication de la demande: 28.05.2014
(73) Titulaire: SEB S.A., 69130 Ecully (FR)
(72) Inventeur: Giraud, Camille, 69001 LYON (FR); Mandica, Franck, 69340 FRANCHEVILLE (FR)
(74) Mandataire: Guéry-Jacques, Géraldine

(56) Documents cités:
- WO-A1-97/22326
- DE-A1-102008 009 472
- US-A- 4 984 568
- US-A1- 2009 062 815

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez les ménages composés de personnes désireuses de soigner leur esthétisme en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant, il est notamment connu la demande de brevet WO2012/140354 qui divulgue un appareil de massage comprenant un corps muni de moyens de motorisation et, une tête de massage activée par les moyens de motorisation. L'appareil de massage comprend également un système de distribution d'un produit cosmétique qui permet de distribuer le produit cosmétique au niveau de la tête de massage tout en contrôlant la quantité de produit délivré, lors de la manipulation et de l'application de la tête de massage sur la peau.

Par ailleurs la demande de brevet WO97/22326 divulgue un appareil tel que décrit dans le préambule de la revendication 1.

La présente invention a pour objet de concevoir un appareil de massage qui offre une plus grande autonomie d'utilisation.

A cet effet, l'invention concerne un appareil de massage comprenant un corps qui comporte des moyens de motorisation, une tête de massage configurée pour être activée par les moyens de motorisation et, un système de distribution d'au moins un produit cosmétique. Cela permet de réaliser un traitement cosmétique en complément du traitement mécanique réalisé par la tête de massage qui travaille la peau en vue d'améliorer la tonicité. Selon l'invention, le système de distribution est configuré pour être actionné manuellement et automatiquement et, comprend un dispositif de débrayage configuré pour basculer le système de distribution soit en mode manuel soit en mode automatique. Ainsi, l'appareil de massage selon l'invention permet de laisser le choix à l'utilisateur de doser lui-même la quantité de produit ou, au contraire, de distribuer une dose de produit prédéterminée par l'appareil et fonction du type de massage réalisé.

Selon l'appareil de massage objet de l'invention, le système de distribution comprend au moins un réservoir de produit cosmétique raccordé à une buse de distribution du produit et au moins un actionneur configuré pour être activé manuellement ou automatiquement et extraire le produit de l'au moins un réservoir lors de son activation. En outre, le dispositif de débrayage est configuré pour permettre le fonctionnement de l'au moins un actionneur soit manuellement soit automatiquement.

Dans un mode de réalisation de l'appareil de massage, l'au moins un réservoir est souple et l'au moins un actionneur comprend une manette et un doigt motorisé configuré pour se déplacer selon un mouvement alternatif. La manette est configurée pour soit être placée dans une première position et presser le réservoir sous une impulsion manuelle soit être placée dans une seconde position et presser le réservoir sous l'impulsion du doigt motorisé.

Dans une variante de réalisation de l'appareil de massage objet de l'invention, l'au moins un réservoir est souple et l'au moins un actionneur comprend un bouton poussoir, une barrette et un système motorisé à crémaillère configuré pour déplacer la barrette sur la longueur du réservoir en pressant sur ce réservoir. Un sélecteur est configuré pour soit être placé dans une première position et presser le réservoir sous une impulsion manuelle du bouton soit être placé dans une seconde position et activer le système motorisé à crémaillère.

Dans une autre variante de réalisation de l'appareil de massage objet de l'invention, l'au moins un réservoir est rigide et l'au moins un actionneur comprend une pompe configurée pour être activée manuellement ou motorisée et un bouton configuré pour soit être placé dans une première position permettant d'activer manuellement la pompe soit placé dans une seconde position permettant d'activer la motorisation de la pompe.

D'autres variantes de réalisation de l'appareil de massage sont envisageables sans sortir du cadre de l'invention, telle que décrit dans les revendication ci-dessous. Selon un mode de réalisation de l'appareil de massage objet de l'invention, le système de distribution de produit cosmétique comprend au moins un réservoir et au moins un actionneur qui sont agencés dans la tête de massage. Cela permet de simplifier la conception de l'appareil de massage et de disposer d'un système de distribution spécifique à la tête de massage. En outre, cela évite la présence d'un tuyau de raccord entre la tête de massage et le corps pour le transfert du produit cosmétique, ce qui améliore l'étanchéité sur l'appareil de massage.

Selon un mode de réalisation de l'appareil de massage objet de l'invention, le système de distribution comprend au moins un réservoir et au moins un actionneur qui sont agencés dans le corps. Cela permet de centraliser les éléments dans le corps de l'appareil de massage et de simplifier la conception des têtes de massage.

Selon un mode de réalisation de l'appareil de massage selon l'invention, le système de distribution comprend au moins un actionneur qui est agencé dans le corps et au moins un réservoir qui est agencé sur la tête de massage. Cela permet de disposer d'un produit cosmétique spécifique à la tête de massage et évite la présence d'un tuyau de raccord entre la tête de massage et le corps pour le transfert du produit cosmétique, ce qui améliore l'étanchéité sur l'appareil de massage.

Selon un mode de réalisation de l'appareil de massage objet de l'invention, le système de distribution comprend au moins un réservoir qui est agencé sur le corps et au moins un actionneur qui est agencé sur la tête. Cela peut présenter pour avantage de disposer d'un plus grand réservoir de produit cosmétique voire de disposer de plusieurs réservoirs avec des produits cosmétiques différents, en fonction du traitement envisagé.

Selon un mode de réalisation de l'appareil de massage objet de l'invention, celui-ci comprend plusieurs types de tête de massage et des moyens d'assemblage configurés pour assembler de manière amovible l'un ou l'autre des types de tête de massage avec le corps. En outre, l'appareil de massage comprend des moyens de reconnaissance du type de tête de massage assemblé sur le corps et des moyens de commande configuré pour activer le système de distribution (5) en fonction du type de tête de massage reconnu. Cela permet d'assembler diverses conceptions de tête de massage et d'adapter au mieux l'appareil de massage en fonction de la tête de massage.

Selon l'appareil de massage objet de l'invention, au moins un type de tête de massage comprend une surface d'application et des embouts de massage qui s'étendent vers l'extérieur de la surface d'application. En outre, le système de distribution est configuré pour éjecter le produit cosmétique sur la surface d'application et/ou au travers des embouts de massage. Ce type de tête de massage permet par exemple de réaliser un pincé simple ou un pincé tournant sur la peau.

Selon l'appareil de massage objet de l'invention, au moins un type de tête de massage (17) comprend deux rouleaux de massage, le système de distribution et les rouleaux étant configurés pour que le produit cosmétique soit transférer sur les surfaces externes des rouleaux de massage. Ce type de tête de massage permet de réaliser un plissage de la peau.

Selon l'appareil de massage objet de l'invention, celui-ci comprend un système de repérage permettant de détecter la quantité de produits restant dans l'au moins un réservoir et/ou lorsque l'au moins un réservoir est vide. Cela permet de réaliser voire d'anticiper le remplacement du réservoir lorsque celui-ci est vide ou presque vide.

Selon l'appareil de massage objet de l'invention, celui-ci comprend un système d'émission d'ondes configuré pour diffuser des ondes au niveau de l'au moins un type de tête de massage en direction de la peau. Ces ondes peuvent être des ondes électromagnétiques, particulièrement lumineuses, visibles ou dans le domaine de l'infrarouge, ou peuvent être sonores par exemple des ultrasons. Cela permet également de travailler de manière complémentaire, la peau de l'utilisateur. Dans un exemple envisagé de réalisation, ces ondes sont lumineuses de couleur rouge ou orange. Dans un mode de réalisation, ce système d'émission d'ondes et un système luminescent configuré pour diffuser de la lumière sur la tête de massage. Cela permet de réaliser un traitement complémentaire à ceux du produit cosmétique et de la tête de massage.

Selon l'appareil de massage objet de l'invention, celui-ci comprend un dispositif de traitement ionophorèse transcutanée qui est configuré pour transmettre sur la peau lors de l'application dudit appareil de massage, un courant permettant d'augmenter et/ou d'accélerer la pénétration d'un produit cosmétique qui est distribué par le système de distribution de produit cosmétique décrit ci-dessus. L'ionophorèse est un traitement qui a été développé initialement pour l'application dans la peau de médicaments, en particulier pour de la médecine sportive, mais est également envisagée maintenant pour une meilleure pénétration d'un cosmétique. Dans un mode de réalisation, ce dispositif de traitement ionophorèse transcutanée comprend deux électrodes à potentiel électrique différent qui peuvent être agencées sur les rouleaux de massage et/ou sur la surface d'application de la tête de massage. Les réglages de l'appareil de massage selon l'invention permettront de réaliser le traitement par ionophorèse transcutanée avant et /ou pendant l'application du produit cosmétique.

La description suivante met en évidence les caractéristiques et avantages de l'appareil de massage selon l'invention, laquelle s'appuie sur des figures, parmi lesquelles :
- La figure 1 schématise un appareil de massage ;
- Les figures 2 et 3 schématisent un appareil de massage comprenant une tête de massage munie de rouleaux de massage ;
- Les figures 4 et 5 schématisent un appareil de massage comprenant une tête de massage munie d'embouts de massage ;
- Les figures 6 et 7 schématisent un appareil de massage comprenant une tête de massage munie de doigts de massage ;
- Les figures 8 et 9 schématisent un appareil de massage comprenant une tête de massage munie d'embouts de massage inclinés ;
- Les figures 10 à 13 schématisent quatre variantes d'agencement d'un système de distribution sur l'appareil de massage ;
- Les figures 14 et 15 schématisent un système de distribution sur l'appareil de massage ;
- Les figures 16 et 17 schématisent une variante de système de distribution sur l'appareil de massage ;
- La figure 18 schématise un système d'assemblage amovible permettant de monter ou démonter rapidement la tête de massage du corps ;
- La figure 19 schématise un système de connexion étanche sur l'appareil de massage équipé d'une tête de massage amovible ;
- La figure 20 schématise un système luminescent sur l'appareil de massage ;
- La figure 21 schématise un dispositif de traitement ionophorèse transcutanée sur l'appareil de massage;
- La figure 22 schématise un système vibratoire sur l'appareil de massage ;
- La figure 23 schématise une peau souple agencée sur la tête de massage ;
- La figure 24 schématise un système de reconnaissance de la tête de massage amovible du corps.

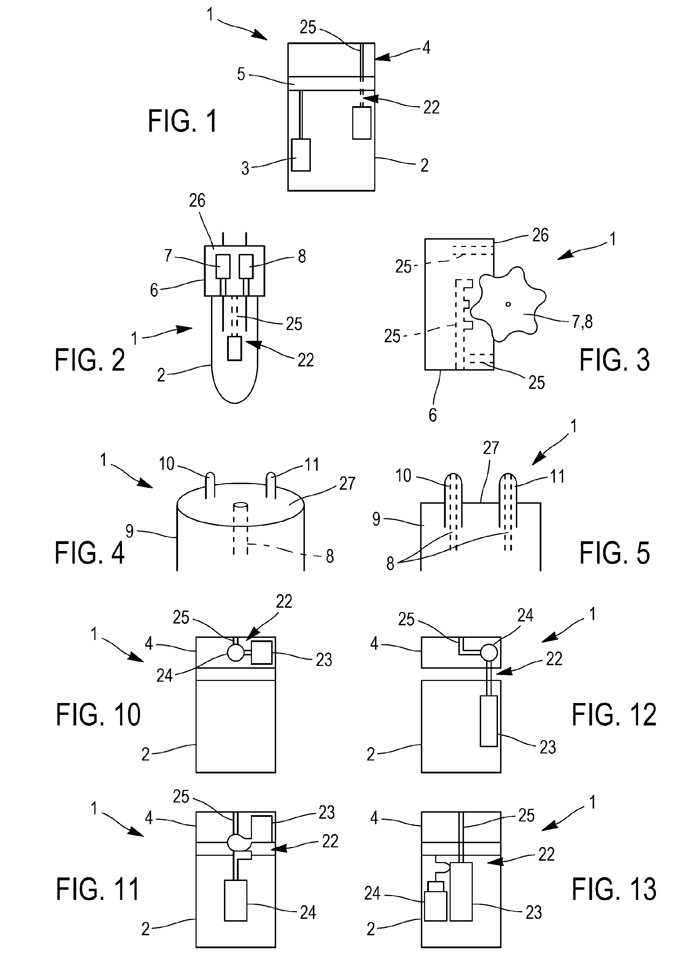

Tel qu'illustré sur la figure 1, l'appareil de massage 1 comprend un corps 2 à l'intérieur duquel est agencé un moteur 3 alimenté électriquement par raccordement soit à une source électrique externe, au moyen d'une prise électrique et d'un transformateur basse tension, soit à source électrique interne du type batterie rechargeable ou piles jetables. L'appareil de massage 1 comprend également un motoréducteur agencé en sortie du moteur 3 pour réduire sa vitesse de rotation et l'adapter au besoin d'utilisation. L'appareil de massage 1 est de préférence configuré pour permettre la connexion de différents types de tête de massage 4. Pour cela, l'appareil de massage comprend des moyens de connexion amovible 5 entre le corps 2 et la tête de massage 4.

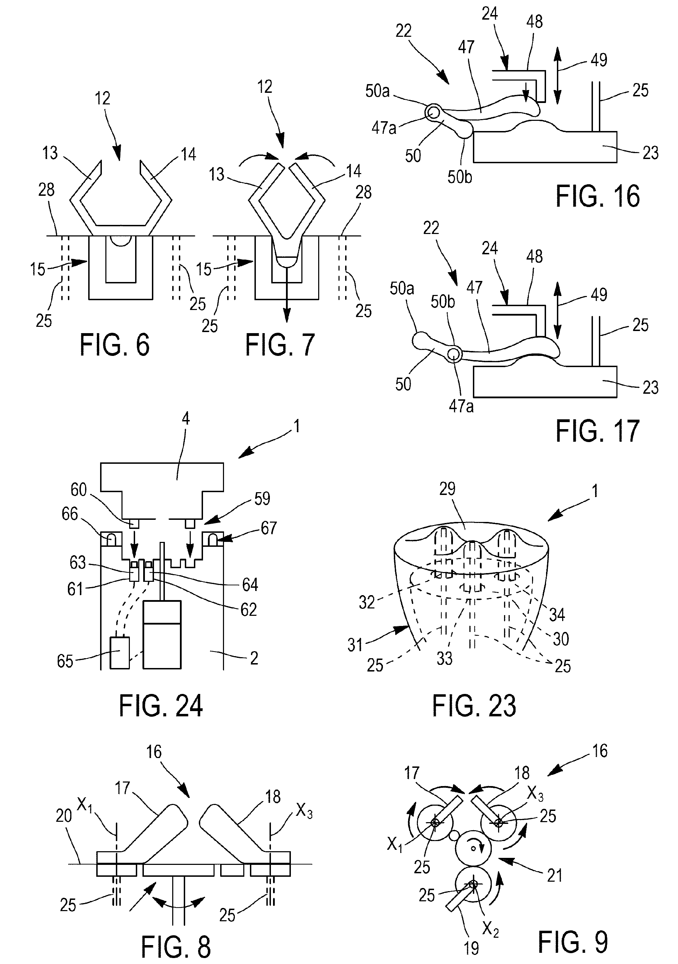

L'appareil de massage 1 selon l'invention peut comprendre différents types de têtes de massage 4. Tel qu'illustré en figure 2 et 3, un premier type de tête de massage 6 comprend deux rouleaux 7, 8. Sur les figures 4 et 5, la tête de massage 9 comprend deux embouts 10, 11, trois ou quatre embouts étant également envisageables. Sur les figures 6 et 7, la tête de massage 12 comprend deux doigts 13, 14 souples actionnés au moyen d'un mécanisme de transmission 15. Sur les figures 8 et 9, la tête de massage 16 comprend trois embouts de massage 17, 18, 19 inclinés par rapport à la surface d'application 20 de la tête de massage 16. Ces embouts 17, 18, 19 sont entraînés par un système d'engrenage 21 assurant leur rotation selon trois axes X₁, X₂, X₃. D'autres variantes de têtes de massage 4 peuvent être envisagées sans sortir du cadre de l'invention.

L'appareil de massage 1 comprend un système de distribution de produit cosmétique 22, schématisé en figure 1 et 10 à 13. Sur la figure 10, le système de distribution 22 comprend un réservoir 23 et un actionneur 24 qui sont agencés à l'intérieur de la tête de massage 4. Sur la figure 11, le système de distribution 22 comprend un réservoir 23 qui est agencé dans la tête de massage 4 et un actionneur 24 qui est agencé dans le corps 2. Sur la figure 12, le système de distribution 22 comprend un réservoir 23 qui est agencé dans le corps et un actionneur 24 qui est agencé dans la tête de massage 4. Sur la figure 13, le système de distribution 22 comprend un réservoir 23 et un actionneur 24 qui sont agencés dans le corps 2. D'autres variantes sont envisageables conformes aux revendications ci-dessous.

L'appareil de massage 1 peut notamment comprendre un système de distribution 22 qui comprend un ou plusieurs réservoirs 23 et un ou plusieurs actionneurs 24 agencés dans la tête de massage 4 et/ou dans le corps. Lorsque plusieurs réservoirs sont agencés, cela permet de réaliser un traitement spécifique parmi plusieurs au moyen d'une même tête de massage 4 voire un traitement spécifique en fonction d'un type de tête de massage 4 assemblé sur le corps 2. De même, la présence de plusieurs actionneurs permet, lorsqu'un ou plusieurs réservoirs sont présents, de pouvoir distribuer du produit cosmétique spécifiquement à un type de tête de massage 4, voire à un emplacement précis parmi d'autres sur une même tête d'assemblage.

L'actionneur 24 agit sur le réservoir 23 pour distribuer du produit cosmétique au travers d'un tuyau 25 illustré sur les figures 1 et 10 à 13. Différentes configurations sont envisageables conformes aux revendications ci-dessous.

Sur les figures 2 et 3, le tuyau 25 peut être agencé pour distribuer le produit cosmétique au niveau de la surface d'application 26 ou directement sur les rouleaux 7, 8. On peut également prévoir un tuyau 25 qui distribue le produit cosmétique à l'intérieur des rouleaux 7, 8. Dans ce cas, les rouleaux 7, 8 sont constitués au moins sur leur surface extérieure d'une matière particulière du type textile micro-encapsulé qui permet de libérer régulièrement en surface des rouleaux 7, 8 du produit cosmétique. Sur les figures 4 et 5, le tuyau 25 permet de distribuer du produit cosmétique en surface 27 de la tête de massage 9 ou au travers des embouts 10, 11. Sur les figures 6 et 7, les tuyaux 25 distribuent du produit cosmétique au niveau de la surface d'application 28. Sur les figures 8 et 9, les tuyaux 25 sont agencés au niveau des axes X₁, X₂, X₃ de rotation des embouts 17, 18, 19. D'autres variantes sont envisageables sans sortir du cadre de l'invention telle que décrite dans les revendications ci-dessous.

Dans une variante illustrée sur la figure 23, l'appareil de massage 1 comprend une peau souple 29 qui est agencée au-dessus de la surface d'application 30 de la tête de massage 31 et recouvre des embouts de massage 32, 33, 34. Cette peau souple 29 évite notamment que les embouts ne paraissent trop agressifs et améliore l'adhérence sur la peau grâce à un contact surfacique. On peut envisager cette peau souple 29 avec les pinces 13, 14 des figures 6 et 7 ou les diverses variantes à embouts, voire d'autres variantes. On constate que les tuyaux 25 passent au travers des embouts de massage 32, 33, 34 et distribue le produit sur la peau souple 29.

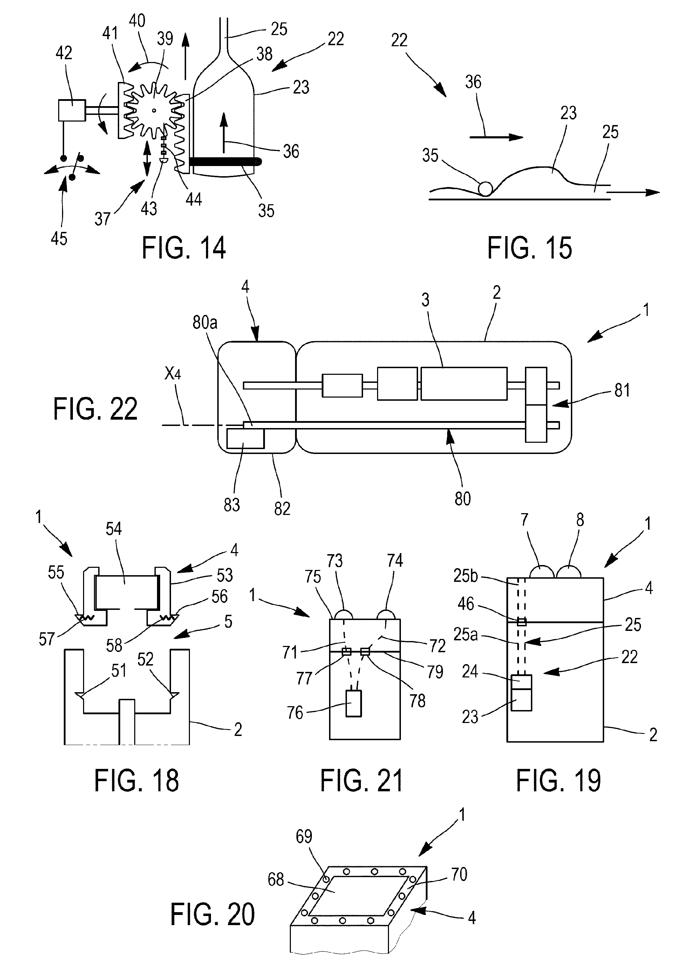

Le système de distribution 22 est configuré pour permettre sont actionnement de manière manuelle ou de manière automatique. Dans la variante illustrée en figures 14 et 15, le système de distribution 22 de produit cosmétique comprend un réservoir de produit 23 souple sur lequel appuie une barrette 35 qui se déplace en translation selon la flèche 36 le long du réservoir de produit 23. Un système d'actionnement 37 permet de déplacer la barrette 35 selon la flèche 36. Ce système d'actionnement 37 est par exemple constitué d'une crémaillère 38 assujettie à la barrette 35. Cette crémaillère 38 engrène avec un pignon 39 qui peut être entraîné en rotation dans le sens de la flèche 40, ce qui permet de translater la crémaillère dans le sens de la flèche 36. L'entraînement du pignon 39 est réalisé soit au moyen d'une roue dentée 41 entraînée par un moteur 42 soit au moyen d'un bouton 43 équipé d'un système de rappel 44 et le pignon 39 comprenant un système anti-retour l'empêchant de tourner dans le sens inverse à la flèche 40. Un sélecteur 45 permet de basculer le système d'actionnement soit en mode automatique pour l'activation de la crémaillère 38 au moyen du moteur 42, soit en mode manuel pour l'activation de la crémaillère 38 au moyen du bouton 43. Le système de distribution 22 comprend un tuyau 25 à l'extrémité du réservoir.

Sur la figure 19, la tête de massage 4 est amovible du corps 2 et le réservoir 23 est disposé dans le corps 2. Dans ce cas, le tuyau 25 comprend une première portion 25a disposée dans le corps 2 de l'appareil de massage et, une seconde portion 25b disposée dans la tête de massage 4. Il est prévu des moyens de raccordement étanche 46 entre la première portion 25a et la seconde portion 25b du tuyau 25. Divers moyens de raccordement étanche 46 pourront être envisagés, par exemple une connexion rapide comprenant une partie mâle et une partie femelle munies de joints d'étanchéité qui s'emboîtent l'une dans l'autre, les joints assurant une étanchéité. D'autres variantes sont envisageables.

Dans une variante illustrée en figures 16 et 17, le système de distribution 22 comprend un réservoir 23 qui est souple et un actionneur 24 qui comprend une manette 47 et un doigt motorisé 48 configuré pour se déplacer selon un mouvement alternatif selon les flèches 49. Le système de distribution 22 comprend un système de glissière 50 qui présente une lumière dans laquelle peut se déplacer l'extrémité arrière 47a de la manette 47. La manette 47 est configurée pour être placée dans une première position illustrée en figure 16, selon laquelle l'extrémité arrière 47a de la manette 47 est positionnée à l'arrière 50a de la lumière 50 et ladite manette 47 est dégagée du doigt motorisé 48. Ainsi, il est nécessaire d'exercer une impulsion manuelle sur la manette 47 pour appuyer sur le réservoir 23 et distribuer du produit cosmétique au travers du tuyau 25. Au contraire, lorsque l'extrémité arrière 47a de la manette 47 est positionnée à l'avant 50b de la lumière 50, tel qu'illustré en figure 17, le doigt motorisé 48 actionne automatiquement la manette 47 qui presse le réservoir 23 et distribue le produit cosmétique au travers du tuyau 25.

Dans une variante, le système de distribution 22 de produit cosmétique peut comprendre un réservoir 23 rigide ou souple et un actionneur 24 qui comprend une pompe raccordée au réservoir 23 et configurée pour être activée manuellement ou automatiquement. Un bouton sélecteur est agencé dans une lumière de manière similaire aux figures 16 et 17 et permet, dans une première position, d'activer manuellement la pompe et, dans une seconde position permettant, d'activer un boîtier de commande qui actionne une motorisation de la pompe.

D'autres variantes de réalisation d'un système de distribution de produit cosmétique avec un système de basculement en mode manuel ou en mode automatique, peuvent être envisagées sans sortir du cadre de l'inventiontelle que décrite dans les revendications ci-dessous.

En complément des systèmes de distribution 23 qui peut fonctionner manuellement ou automatiquement, à titre d'exemple non compris dans l'invention, le produit cosmétique étant diffusé régulièrement grâce à des phénomènes physiques ne nécessitant pas un actionnement extérieur.

Tel qu'illustré en figures 1 et 18, l'appareil de massage 1 comprend des moyens d'assemblage 5 qui sont configurés pour que la tête de massage 4 puisse être montée et démontée rapidement du corps 2. Ces moyens d'assemblage 5 sont mis en oeuvre au moyen d'un système de fixation amovible entre la tête de massage et le corps. Selon le mode de réalisation de la figure 18, le corps 2 comprend des encoches 51, 52 et la tête de massage 4 comprend une pièce 53 qui reçoit le mécanisme de transmission 54 du mouvement aux éléments de massage, connecté au moteur 3 par le biais d'un système de connexion qui, de préférence, permet de s'affranchir de l'angle d'inclinaison de la tête de massage 4 par rapport au corps 2. Cette pièce 53 comprend des crans 55, 56 et des moyens de rétractation 57, 58 des crans 55, 56 permettant de les escamoter pour leur positionnement dans les encoches 51, 52 et aussi leur retrait de ces encoches 51, 52.

Différents systèmes d'encliquetage ou moyens de rétractation peuvent être envisagés pour maintenir assembler une tête de massage 4 sur le corps 2. On peut envisager un système de butée, un système à baïonnette, un système à clapet et ressort ou, un système de blocage avec un cran et un loquet de déblocage. Ces différentes variantes de réalisation ne sont pas limitatives.

Tel qu'illustré en figure 24, l'appareil de massage 1 comprend des moyens de reconnaissance 59 de la tête de massage 4 assemblée sur le corps 2. La tête de massage 4 comprend une protubérance 60 de forme circulaire qui est prévue pour être engagée dans une rainure circulaire 61 sur le corps 2. Le corps 2 comprend une seconde rainure circulaire 62 pouvant réceptionner une seconde protubérance similaire à la protubérance 60, mais de diamètre correspondant à la rainure circulaire 62. Dans ces rainures circulaires 61, 62 sont agencés des contacteurs mécaniques 63, 64. Lorsque la tête de massage 4 est assemblée sur le corps 2, la protubérance 60 actionne le contacteur mécanique 63. Différentes têtes de massage peuvent être assemblées sur le corps 2 et comprendre une ou deux des protubérances 60 précitées. Lors de l'assemblage de ces têtes de massage sur le corps 2, celle actionnent l'un et/ou l'autre des deux contacteurs mécaniques 63, 64, ce qui permet leur reconnaissance en fonction du codage binaire programmé sur un boîtier de commande 65 illustré en figure 24. Ce boîtier de commande 65 est configuré pour contrôler les différents actionneurs présents dans l'appareil de massage 1 en fonction du type de tête de massage assemblée. Ainsi, par exemple, le boîtier de commande 161 permet de contrôler l'actionnement des moteurs, des pompes de distribution de produit cosmétique et autre.

Des variantes sont envisageables dans le cadre de l'invention telle que décrite dans les revendications ci-dessous.

On peut notamment remplacer les contacteurs mécaniques par des contacteurs électriques ; des contacteurs optiques ou des capteurs magnétiques. Sur la figures 24 sont représentés des capteurs optiques 66, 67.

D'autres variantes sont envisageables sans sortir du cadre de l'invention telle que décrite dans les revendications ci-dessous. Notamment quant à la reconnaissance des têtes de massage sur le corps 2 et au boîtier de commande 65. Les capteurs pourront par exemple permettre de détecter l'assemblage convenable de la tête de massage 147 sur le corps 2.

Dans une variante illustrée en figure 20, l'appareil de massage 1 comprend au niveau de la surface d'application 68 sur la tête de massage 4, des diodes luminescentes 69 qui sont commandées par un boîtier électronique (non illustré) agencé à l'intérieur du corps 2. Ces diodes luminescentes 69 pourront être allumées soit automatiquement lors de l'actionnement de la tête de massage 4, soit séparément au moyen d'un bouton de commande distinct (non illustré). Les diodes luminescentes 69 seront disposées par exemple sur le bord périphérique 70 de la surface d'application 68, voire réparties sur ladite surface d'application 68 en dehors des trajectoires des éléments de massage. On pourra utiliser des diodes luminescentes de différentes couleur ou multi-couleurs, selon la longueur d'onde souhaitée et/ou le traitement recherché, voire prévoir un boîtier de commande permettant de modifier la longueur d'onde de ces diodes luminescentes 69. Un système de transmission de la lumière est agencé entre la tête de massage 4 et le corps, lorsque cette tête de massage 4 est amovible du corps 2. Ce système de transmission de la lumière comprend une connexion par fibres optiques qui permet de diriger la lumière émise vers la surface d'application 68 ou sur les éléments de massage tels que des embouts de massage.

Dans une variante de réalisation illustrée en figure 21, l'appareil de massage 1 comprend deux électrodes 71, 72 qui présentent un potentiel électrique différent. Ces électrodes 71, 72 peuvent consister en deux éléments de massage 73, 74 ou être agencées sur la surface d'application 75. Ces électrodes 111, 112 sont alimentées par une source électrique 76 agencé dans le corps 2. Cette conception permet de réaliser un traitement par inophorèse transcutanée avant ou pendant l'application d'un produit cosmétique sur la peau, ce qui permet d'accélérer la pénétration du produit cosmétique. Ce produit cosmétique peut être distribué de manière naturelle, manuelle ou automatique par l'appareil de massage 1, voire appliqué directement sur la peau par l'utilisateur. L'appareil de massage 1 comprend des moyens de connexion amovibles 77, 78 qui permettent de déconnecter ou de connecter rapidement les électrodes 71, 72 lors du démontage ou du montage de la tête de massage 4 sur le corps 2. On peut par exemple prévoir des contacteurs électriques au niveau de la zone d'assemblage 79 entre la tête de massage 4 et le corps 2.

Tel qu'illustré en figure 22, l'appareil de massage 1 comprend un arbre 80 qui est entraîné en rotation selon un axe X₄ par le moteur 3, au moyen d'un système d'engrenage 81 connu de l'homme du métier. L'extrémité 80a de cet arbre 80 est agencée dans la tête de massage 4, par exemple sur le carter 82 ou sur les éléments de massage, et reçoit une masselotte 83 qui tourne en balourd autour de l'axe X₄ et permet de faire vibrer la tête de massage 4 durant l'application de l'appareil de massage 1. D'autres variantes de système vibratoire restent envisageables.

Le boîtier de commande 65 est également configuré pour contrôler les diodes luminescentes 69, la source électrique 76 commandant les électrodes 71, 72 et le système vibratoire, en fonction de la reconnaissance de la tête de massage 4 assemblée sur le corps.

D'autres caractéristiques sont envisageables dans le cadre de l'invention telle que décrite dans les revendications ci-dessous. On pourra en particulier envisager des variantes de système de distribution 22 de produit cosmétique configurées pour être actionnée soit manuellement soit automatiquement.

L'appareil de massage 1 comprend également un système de repérage ou de détection permettant de détecter la quantité de produit dans l'au moins réservoir 23 et/ou lorsque l'au moins un réservoir est vide. Ce système de repérage peut par exemple être un capteur de position qui pour le mode de conception illustré en figures 14 et 15, détecteur le fin de course de la barrette 35. On peut également envisager que ce système de repérage ou de détection soit une minuterie qui compte le temps d'activation de l'actionneur 24 et se déclenche lorsqu'une durée définie est atteinte.

## Revendications

1. Appareil de massage (1) comprenant un corps (2) qui comporte des moyens de motorisation (3) et une tête de massage (4) configurée pour être activée par les moyens de motorisation et un système de distribution (22) d'au moins un produit cosmétique configuré pour être actionné manuellement et automatiquement, **caractérisé en ce que** le système de distribution comprend un dispositif de débrayage configuré pour basculer le système de distribution soit en mode manuel soit en mode automatique.

2. Appareil de massage (1) selon la revendication 1, dans lequel le système de distribution (22) comprend au moins un réservoir (23) de produit cosmétique raccordé à une buse de distribution (25) du produit et au moins un actionneur (24) configuré pour être activé manuellement ou automatiquement et extraire le produit de l'au moins un réservoir lors de son activation et, le dispositif de débrayage étant configuré pour permettre le fonctionnement de l'au moins un actionneur soit manuellement soit automatiquement.

3. Appareil de massage (1) selon la revendication 2, dans lequel l'au moins un réservoir (23) est souple et l'au moins un actionneur (24) comprend une manette (47) et un doigt motorisé (48) configuré pour se déplacer selon un mouvement alternatif, la manette étant configurée pour soit être placée dans une première position et presser le réservoir sous une impulsion manuelle soit être placée dans une seconde position et presser le réservoir sous l'impulsion du doigt motorisé.

4. Appareil de massage (1) selon la revendication 2, dans lequel l'au moins un réservoir (23) est souple et l'au moins un actionneur (24) comprend un bouton poussoir (43), une barrette (35) et un système motorisé à crémaillère (38) configuré pour déplacer la barrette sur la longueur du réservoir en pressant sur ce réservoir, un sélecteur (45) étant configuré pour soit être placé dans une première position et presser le réservoir sous une impulsion manuelle du bouton soit être placé dans une seconde position et activer le système motorisé à crémaillère.

5. Appareil de massage (1) selon la revendication 2, dans lequel l'au moins un réservoir (23) est rigide ou souple et l'au moins un actionneur (24) comprend une pompe configurée pour être activée manuellement ou motorisée et un bouton configuré pour soit être placé dans une première position permettant d'activer manuellement la pompe soit placé dans une seconde position permettant d'activer la motorisation de la pompe.

6. Appareil de massage (1) selon l'une des revendications 2 à 5, dans lequel au moins un réservoir (23) et au moins un actionneur (24) sont agencés dans la tête de massage (4).

7. Appareil de massage (1) selon l'une des revendications 2 à 5, dans lequel au moins un réservoir (23) et au moins un actionneur (24) sont agencés dans le corps (2).

8. Appareil de massage (1) selon l'une des revendications 2 à 5, dans lequel au moins un actionneur (24) est agencé dans le corps (2) et au moins un réservoir (23) est agencé sur la tête de massage (4).

9. Appareil de massage (1) selon l'une des revendications 2 à 5, dans lequel au moins un réservoir (23) est agencé sur le corps (2) et au moins un actionneur (24) est agencé sur la tête (4).

10. Appareil de massage (1) selon l'une des revendications 1 à 9, lequel comprend plusieurs types de tête de massage (4), des moyens d'assemblage (5) configurés pour assembler de manière amovible l'un ou l'autre des types de tête de massage avec le corps (2), des moyens de reconnaissance (59) du type de tête de massage (4) assemblé sur le corps et des moyens de commande (65) configuré pour activer le système de distribution (22) en fonction du type de tête de massage reconnu.

11. Appareil de massage (1) selon l'une des revendications 1 à 10, dans lequel au moins un type de tête de massage (4, 9) comprend une surface d'application et des embouts de massage (10, 11) qui s'étendent vers l'extérieur de la surface d'application, le système de distribution étant configuré pour éjecter le produit cosmétique sur la surface d'application et/ou au travers des embouts de massage.

12. Appareil de massage (1) selon l'une des revendications 1 à 10, dans lequel au moins un type de tête de massage (4, 6)) comprend deux rouleaux de massage (7, 8), le système de distribution et les rouleaux étant configurés pour que le produit cosmétique soit transféré sur les surfaces externes des rouleaux de massage.

13. Appareil de massage (1) selon l'une des revendications 1 à 12, lequel comprend un système de repérage permettant de détecter la quantité de produit dans l'au moins réservoir (23) et/ou lorsque l'au moins un réservoir est vide.

14. Appareil de massage selon l'une des revendications 1 à 13, lequel comprend un système d'émission d'onde (69) configuré pour diffuser des ondes au niveau de la tête de massage (4) en direction de la peau.

15. Appareil de massage (1) selon l'une des revendications 1 à 14, lequel comprend un dispositif de traitement ionophorèse transcutanée (71, 72, 76) qui est configuré pour transmettre sur la peau lors de l'application dudit appareil de massage, un courant permettant d'augmenter et/ou d'accélérer la pénétration d'un produit cosmétique.

## Patentansprüche

1. Massagegerät (1) mit einem Körper (2), der Antriebsmittel (3) und einen Massagekopf (4), der dafür ausgelegt ist, von den Antriebsmitteln aktiviert zu werden, und ein Abgabesystem (22) zur Abgabe von zumindest einem Kosmetikprodukt umfasst, das dafür ausgelegt ist, manuell oder automatisch betätigt zu werden, **dadurch gekennzeichnet, dass** das Abgabesystem eine Vorrichtung zum Entkuppeln umfasst, das dafür ausgelegt ist, das Abgabesystem entweder in den manuellen Modus oder in den Automatikmodus zu bewegen.

2. Massagegerät (1) nach Anspruch 1, wobei das Abgabesystem (22) zumindest einen Behälter (23) mit einem Kosmetikprodukt umfasst, der mit einer Abgabedüse (25) zur Abgabe des Produkts und zumindest einem Betätigungselement (24) verbunden ist, das dafür ausgelegt ist, manuell oder automatisch betätigt zu werden und bei seiner Betätigung das Produkt aus zumindest einem Behälter zu entnehmen, wobei die Vorrichtung zum Entkuppeln dafür ausgelegt ist, entweder den manuellen oder den automatischen Betrieb zumindest eines Betätigungselements zu ermöglichen.

3. Massagegerät (1) nach Anspruch 2, wobei zumindest ein Behälter (23) flexibel ist und wobei zumindest ein Betätigungselement (24) einen Hebel (47) und einen motorisierten Finger (48) umfasst, der dafür ausgelegt ist, sich hin- und herzubewegen, wobei der Hebel dafür ausgelegt ist, entweder in eine erste Position gebracht zu werden und den Behälter durch einen manuellen Impuls zusammenzudrücken oder in eine zweite Position gebracht zu werden und den Behälter durch einen Impuls des motorisierten Fingers zusammenzudrücken.

4. Massagegerät (1) nach Anspruch 2, wobei zumindest ein Behälter (23) flexibel ist und wobei zumindest ein Betätigungselement (24) einen Druckknopf (43), eine Leiste (35) und einen Zahnstangenantrieb (38) umfasst, der dafür ausgelegt ist, die Leiste über die Länge des Behälters zu verschieben und gleichzeitig diesen zusammenzudrücken, wobei ein Wahlhebel (45) dafür ausgelegt ist, entweder in eine erste Position gebracht zu werden und den Behälter durch einen manuellen Impuls des Knopfes zusammenzudrücken oder in eine zweite Position gebracht zu werden und den Zahnstangenantrieb zu aktivieren.

5. Massagegerät (1) nach Anspruch 2, wobei zumindest ein Behälter (23) starr oder flexibel ist und wobei zumindest ein Betätigungselement (24) eine Pumpe, die dafür ausgelegt ist, manuell oder von einem Motor betätigt zu werden, und einen Knopf umfasst, der dafür ausgelegt ist, entweder in eine erste Position gebracht zu werden, welche die manuelle Betätigung der Pumpe ermöglicht, oder in eine zweite Position gebracht zu werden, welche die Betätigung des Antriebs der Pumpe ermöglicht.

6. Massagegerät (1) nach einem der Ansprüche 2 bis 5, wobei zumindest ein Behälter (23) und zumindest ein Betätigungselement (24) in dem Massagekopf (4) angeordnet sind.

7. Massagegerät (1) nach einem der Ansprüche 2 bis 5, wobei zumindest ein Behälter (23) und zumindest ein Betätigungselement (24) in dem Körper (2) angeordnet sind.

8. Massagegerät (1) nach einem der Ansprüche 2 bis 5, wobei zumindest ein Betätigungselement (24) in dem Körper (2) angeordnet ist und zumindest ein Behälter (23) auf dem Massagekopf (4) angeordnet ist.

9. Massagegerät (1) nach einem der Ansprüche 2 bis 5, wobei zumindest ein Behälter (23) in dem Körper (2) angeordnet ist und zumindest ein Betätigungselement (24) auf dem Massagekopf (4) angeordnet ist.

10. Massagegerät (1) nach einem der Ansprüche 1 bis 9, das mehrere Arten von Massageköpfen (4), Montagemittel (5), die dafür ausgelegt sind, die verschiedenen Arten von Massageköpfen abnehmbar an dem Körper (2) zu montieren, Erkennungsmittel (59) zum Erkennen der Art des an den Körper montierten Massagekopfes (4) und Bedienelemente (65) umfasst, die dafür ausgelegt sind, das Abgabesystem (22) in Abhängigkeit von der Art des erkannten Massagekopfes zu aktivieren.

11. Massagegerät (1) nach einem der Ansprüche 1 bis 10, wobei zumindest eine Art von Massagekopf (4, 9) eine Applikationsfläche und Massagespitzen (10, 11) umfasst, die sich von der Applikationsfläche nach außen erstrecken, wobei das Abgabesystem dafür ausgelegt ist, das Kosmetikprodukt auf die Applikationsfläche und/oder durch die Massagespitzen auszustoßen.

12. Massagegerät (1) nach einem der Ansprüche 1 bis 10, wobei zumindest eine Art von Massagekopf (4, 6) zwei Massagerollen (7, 8) umfasst, wobei das Abgabesystem und die Rollen dafür ausgelegt sind, das Kosmetikprodukt auf die Außenflächen der Massagerollen gelangen zu lassen.

13. Massagegerät (1) nach einem der Ansprüche 1 bis 12, das ein Bestimmungssystem umfasst, mit dem sich die in zumindest einem Behälter (23) befindliche Produktmenge erkennen lässt und/oder das erkennen lässt, wenn zumindest ein Behälter leer ist.

14. Massagegerät nach einem der Ansprüche 1 bis 13, das ein System zur Emission von Wellen (69) umfasst, das dafür ausgelegt ist, am Massagekopf (4) in Richtung der Haut Wellen auszusenden.

15. Massagegerät (1) nach einem der Ansprüche 1 bis 14, das eine Vorrichtung zur transkutanen Behandlung durch lontophorese (71, 72, 76) umfasst, die dafür ausgelegt ist, bei Anwendung des genannten Massagegerätes einen Strom auf die Haut zu leiten, durch den sich das Eindringen eines Kosmetikprodukts erhöhen und/oder beschleunigen lässt.

## Claims

1. Massage apparatus (1) comprising a body (2) having drive means (3) and a massage head (4) configured to be activated by the drive means and a system (22) for dispensing at least a cosmetic product configured to be actuated manually and automatically, **characterised in that** the dispensing system comprises a clutch device configured to switch the dispensing system either into manual mode or automatic mode.

2. Massage apparatus (1) according to claim 1, wherein the dispensing system (22) comprises at least one tank (23) of cosmetic product connected to a nozzle (25) for dispensing the product and at least one actuator (24) configured to be activated manually or automatically and extract the product from the at least one tank when activated and, the clutch device being configured to allow the operation of the at least one actuator, either manually or automatically.

3. Massage apparatus (1) according to claim 2, wherein the at least one tank (23) is flexible and the at least one actuator (24) comprises a handle (47) and a motorised pin (48) configured to move in a reciprocating motion, the handle being configured to be placed either in a first position and press the tank under a manual pulse or to be placed in a second position and press the tank under the pulse of the motorised pin.

4. Massage apparatus (1) according to claim 2, wherein the at least one tank (23) is flexible and the at least one actuator (24) comprises a push button (43) a bar (35) and a motorised rack system (38) configured to move the bar over the length of the tank by pressing on this tank, a selector (45) being configured to be placed either in a first position and press the tank under a manual pulse of the button or to be placed in a second position and activate the motorised rack system.

5. Massage apparatus (1) according to claim 2, wherein the at least one tank (23) is rigid or flexible and the at least one actuator (24) comprises a pump configured to be activated manually or motorised and a button configured to be placed either in a first position to manually activate the pump or placed in a second position to activate the pump drive.

6. Massage apparatus (1) according to one of claims 2 to 5, wherein at least one tank (23) and at least one actuator (24) are arranged in the massage head (4).

7. Massage apparatus (1) according to one of claims 2 to 5, wherein at least one tank (23) and at least one actuator (24) are arranged in the body (2).

8. Massage apparatus (1) according to one of claims 2 to 5, wherein at least one actuator (24) is arranged in the body (2) and at least one tank (23) is arranged on the massage head (4).

9. Massage apparatus (1) according to one of claims 2 to 5, wherein at least one tank (23) is arranged on the body (2) and at least one actuator (24) is arranged on the head (4).

10. Massage apparatus (1) according to one of claims 1 to 9, which comprises several types of massage head (4), assembly means (5) configured to removably assemble one of the types of massage head with the body (2), means (59) for recognising the type of massage head (4) assembled on the body and control means (65) configured to activate the dispensing system (22) depending on the type of massage head recognised.

11. Massage apparatus (1) according to one of claims 1 to 10, wherein at least one type of massage head (4, 9) comprises an application surface and massaging tips (10, 11) extending towards the outside of the application surface, the dispensing system being configured to eject the cosmetic product on the application surface and/or through the massaging tips.

12. Massage apparatus (1) according to one of claims 1 to 10, wherein at least one type of massage head (4, 6) comprises two massaging rollers (7, 8), the dispensing system and the rollers being configured so that the cosmetic product is transferred to the outer surfaces of the massaging rollers.

13. Massage apparatus (1) according to one of claims 1 to 12, which comprises a detection system for detecting the amount of product in the at least one tank (23) and/or when the at least one tank is empty.

14. Massage apparatus according to one of claims 1 to 13, which comprises a wave transmission system (69) configured to emit waves from the massage head (4) towards the skin.

15. Massage apparatus (1) according to one of claims 1 to 14, which comprises a transcutaneous ionophoresis treatment device (71, 72, 76) which is configured to transmit to the skin during application of said massage apparatus, a current used to increase and/or accelerate penetration of a cosmetic product.
